(19) Europäisches Patentamt
European Patent Office
Office européen des brevets

(11) Publication number: **0 129 908**
**B1**

(12) # EUROPEAN PATENT SPECIFICATION

(45) Date of publication of patent specification: **13.05.87**

(51) Int. Cl.4: **A 61 K 31/557**

(21) Application number: **84107316.6**

(22) Date of filing: **26.06.84**

(54) **Use of misoprostol to manufacture cholesterol lowering medicines.**

(30) Priority: **27.06.83 US 508373**

(43) Date of publication of application:
**02.01.85 Bulletin 85/01**

(45) Publication of the grant of the patent:
**13.05.87 Bulletin 87/20**

(84) Designated Contracting States:
**AT BE CH DE FR GB IT LI NL SE**

(56) References cited:
**US-A-3 965 143**
**US-A-4 301 146**

**CHEMICAL ABSTRACTS, vol. 84, 1976, page 86, abstract no. 145224v Columbus, Ohio (US) B. JACOTOT et al.: "Effect of prostaglandin PGE1 on blood and tissue lipids in experimental atherosclerosis in rabbit".**

(73) Proprietor: **G.D. Searle & Co.**
**P.O. Box 1045**
**Skokie Illinois 60076 (US)**

(72) Inventor: **Esam, Zafer Dajani**
**Long Grove**
**Illinois 60047 (US)**

(74) Representative: **Wolff, Hans Joachim, Dr.jur. Dipl.-Chem.**
**Beil, Wolff & Beil Rechtsanwälte Postfach 80 01 40 Adelonstrasse 58**
**D-6230 Frankfurt am Main 80 (DE)**

**EP 0 129 908 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

Prostaglandins having the structure

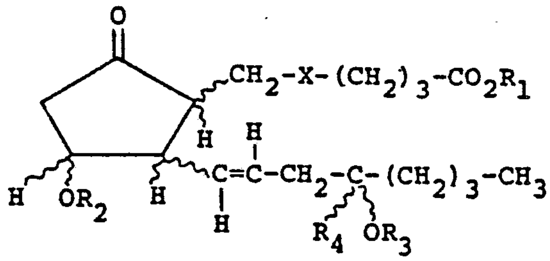

wherein $R_1$ is hydrogen or lower alkyl having 1—4 carbon atoms; $R_2$ and $R_3$ is hydrogen or lower alkanoyl having 1—4 carbon atoms $R_4$ is lower alkyl having 1—4 carbon atoms, and X is $—CH_2—CH_2—$ or $—CH{=}CH—$ are known compounds having utility in the treatment of peptic ulcer disease. The compounds, their preparation, and use in the treatment of ulcers are described in U.S. patents 3,965,143, 4,060,691 and 4,087,621. Jacotot and Gero (Path Biol *23* 711—715 (1975)) describe the effect of prostaglandin $PGE_1$ on blood and tissue lipids in rabbits experimental atherosclerosis.

The 1:1 mixture of 16-methyl-16-hydroxy prostaglandins of formulas I and II used in this invention lower elevated cholesterol levels to normal levels in patients with hypercholesterol levels to normal levels in patients with hypercholesterolemia and without effectively lowering normal cholesterol levels. Thus a new medical use of a known mixture has been discovered.

DESCRIPTION OF THE INVENTION

This invention encompasses the use of a 1:1 mixture of prostaglandins of the formulas

(I) (±) 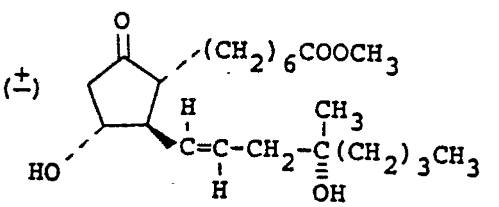 (±) 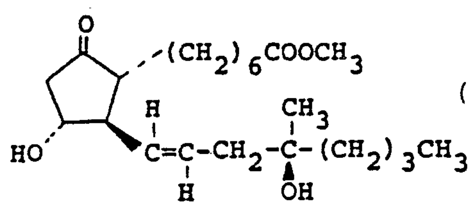 (II)

for the manufacture of medicines for lowering cholesterol levels in humans having elevated cholesterol level.

The 1:1 mixture of prostaglandins of formulas I and II contains four stereoisomers. The USAN (United States Adopted Name) name for this mixture is misoprostol. It originaly is a drug developed for the treatment of peptic ulcer disease. In manufacturing the medicines the mixture may be combined with a variety of pharmaceutically acceptable carriers and administered in a variety of dosage forms such as pills, tablets and preformulated liquids as well as sustained release dosage forms.

The preferred dosage is 100—200 micrograms orally four times per day. Dosages from 1/10 to five (5) times the 100 micrograms — four times daily dosage are effective cholesterol lowering doses.

Example 1

Three patients with elevated cholesterol levels indicated below were given a 100 micrograms of misoprostol four times daily as indicated below:

| Patient | Pre-Study | mg/dl cholesterol After-Study |
|---|---|---|
| 07(CSO) | 333 | 279 (4 weeks) |
| 15(SH) | 353 | 268 (2 weeks) |
| 18(FJ) | 281 | 203 (2 weeks) |

The normal range for cholesterol is 140—270 milligrams/deciliter (mg/dl).

**Claim**

Use of a 1:1 mixture of prostaglandins of the formulas

(±) 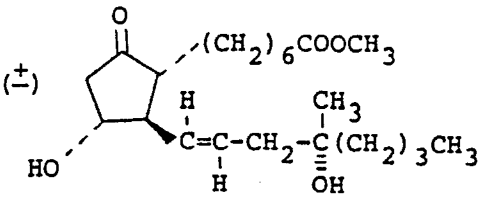 (±) 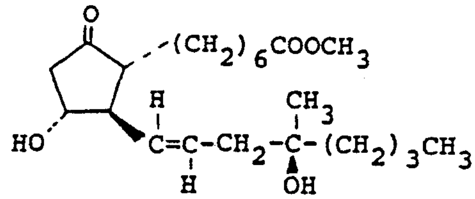

for the manufacture of medicaments for lowering cholesterol levels in humans.

**Patentanspruch**

Verwendung einer 1:1-Mischung von Prostaglandinen der Formeln

(±) 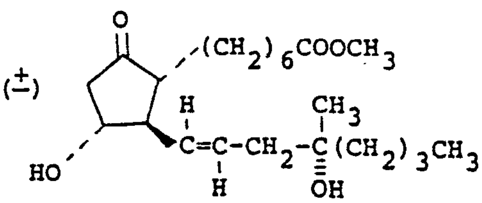 (±) 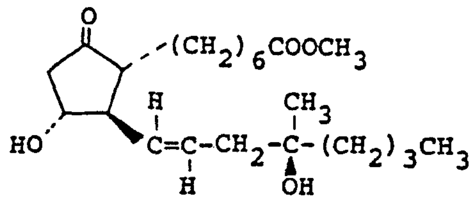

zur Herstellung von Arzneimitteln zur Absenkung des Cholesterinspiegels bei Menschen.

**Revendication**

Utilisation d'un mélange 1:1 de prostaglandines de formules

(±) 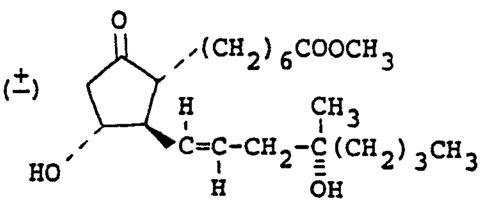 (±) 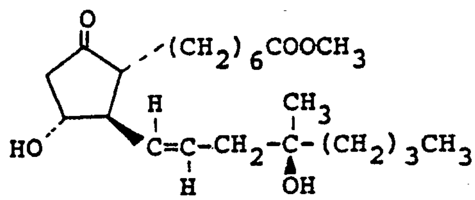

pour la fabrication de médicaments pour abaisser les taux de cholestérol chez les humains.